(19) Europäisches Patentamt
European Patent Office
Office européen des brevets

(11) **EP 4 623 815 A1**

(12) **EUROPEAN PATENT APPLICATION**

(43) Date of publication:
**01.10.2025  Bulletin 2025/40**

(21) Application number: **25164351.6**

(22) Date of filing: **18.03.2025**

(51) International Patent Classification (IPC):
*A61B 5/0205* (2006.01)    *A61B 5/00* (2006.01)
*A61B 5/024* (2006.01)     *A61B 5/08* (2006.01)
*A61B 5/113* (2006.01)     *B60N 2/00* (2006.01)

(52) Cooperative Patent Classification (CPC):
**A61B 5/7207; A61B 5/0205; A61B 5/024;
A61B 5/0816; A61B 5/113; A61B 5/6893;
A61B 5/725; A61B 5/7257; B60N 2/0022;**
A61B 5/0077; A61B 2562/0247

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**GE KH MA MD TN**

(30) Priority:  **29.03.2024  JP 2024057309**

(71) Applicant: **Aisin Corporation
Kariya, Aichi 448-8650 (JP)**

(72) Inventors:
• **TSUCHIYA, Kento
Kariya, Aichi, 448-8650 (JP)**
• **NOMA, Hiroshi
Kariya, Aichi, 448-8650 (JP)**

(74) Representative: **TBK
Bavariaring 4-6
80336 München (DE)**

(54) **BIOLOGICAL INFORMATION DETECTION DEVICE**

(57)    A biological information detection device (111) includes: a sensor (12) configured to detect information on a body motion of a person; a frequency analysis unit (202) configured to perform frequency analysis on a detection signal of the information on the body motion detected by the sensor; a filter processing unit (205) configured to generate a filter based on a result of the frequency analysis and apply the generated filter to the detection signal; and a detection unit (206) configured to detect biological information that is a heartbeat interval based on a detection signal before applying the filter corresponding to a peak of a detection signal after applying the filter.

*FIG. 4*

## Description

TECHNICAL FIELD

This disclosure relates to a biological information detection device.

BACKGROUND DISCUSSION

In the related art, a technique of acquiring a body motion of a person and detecting biological information is known. For example, JP 2009-22638A (Reference 1) discloses a technique of extracting a heartbeat waveform by inverse conversion of a heartbeat signal in a passband by using a filter in which a maximum peak in a section of 0.5 Hz to 2 Hz is set as a fundamental frequency and a harmonic band up to a fourth harmonic which is a frequency four times the fundamental frequency is set as a passband by frequency conversion.

However, in the related art, when noise is just superimposed on the harmonic band or when an intensity of a signal of a specific harmonic component is insufficient due to physical constitution or contact with a sensor, a signal-to-noise ratio of a signal waveform after filter application deteriorates. In the related art, when noise is superimposed near the fundamental frequency, the fundamental frequency cannot be correctly estimated. Therefore, in the related art, it may be difficult to detect biological information such as a heartbeat with high accuracy.

A need thus exists for a biological information detection device and a biological information detection method that are capable of detecting biological information with high accuracy.

SUMMARY

A biological information detection device according to the present disclosure includes: a sensor configured to detect information on a body motion of a person; a frequency analysis unit configured to perform frequency analysis on a detection signal of the information on the body motion detected by the sensor; a filter processing unit configured to generate a filter based on a result of the frequency analysis and apply the generated filter to the detection signal; and a detection unit configured to detect biological information that is a heartbeat interval based on a detection signal before applying the filter corresponding to a peak of a detection signal after applying the filter.

According to a biological information detection device according to this disclosure, it is possible to detect biological information with high accuracy.

BRIEF DESCRIPTION OF THE DRAWINGS

The foregoing and additional features and characteristics of this disclosure will become more apparent from the following detailed description considered with the reference to the accompanying drawings, wherein:

FIG. 1 is a diagram showing an example of a configuration of a vehicle according to a first embodiment;
FIG. 2 is a diagram showing an example of a configuration of a pneumatic sensor according to the first embodiment;
FIG. 3 is a block diagram showing an example of a system configuration and a hardware configuration of the biological information detection system according to the first embodiment;
FIG. 4 is a diagram showing an example of a functional configuration of the biological information detection device according to the first embodiment;
FIG. 5 is a flowchart showing an example of a procedure of biological information detection processing according to the first embodiment;
FIG. 6 is a diagram showing an example of a waveform of a frequency analysis result according to the first embodiment;
FIG. 7 is a flowchart showing an example of a procedure of fundamental frequency estimation processing according to the first embodiment;
FIG. 8 is a flowchart showing an example of a procedure of filter processing according to the first embodiment;
FIG. 9 is a flowchart showing an example of a procedure of peak detection processing according to the first embodiment;
FIG. 10 is a diagram showing a comparison between a detection signal before filter application and a detection signal after filter application according to the first embodiment;
FIG. 11 is a flowchart showing an example of a procedure of peak correction processing according to the first embodiment;
FIG. 12 is a flowchart showing an example of a procedure of biological information detection processing according to a second embodiment; and
FIG. 13 is a flowchart showing an example of a procedure of fundamental frequency estimation processing according to the second embodiment.

DETAILED DESCRIPTION

Hereinafter, embodiments disclosed here will be described. Configurations of embodiments to be described below, and operations, results, and effects of the configurations are examples. The embodiments disclosed here can be implemented by configurations other than the configurations disclosed in the embodiments to be described below, and at least one of various effects based on a basic configuration and a derivative effect can be obtained.

First Embodiment

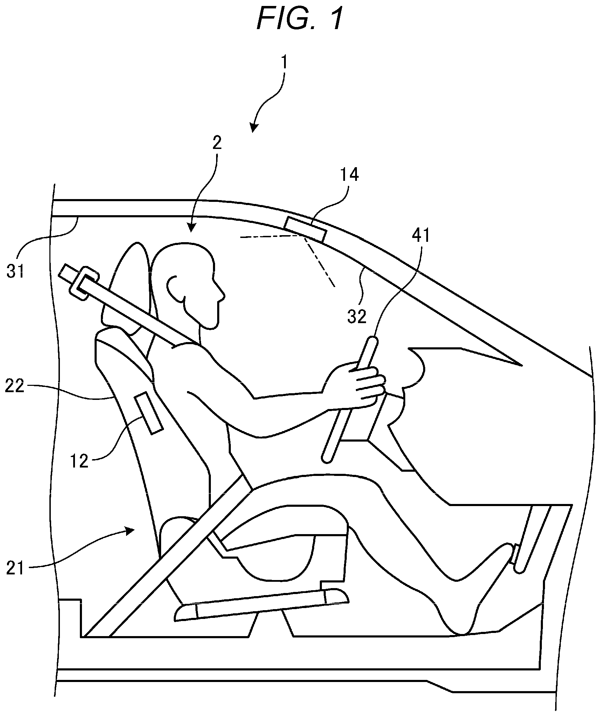

FIG. 1 is a diagram showing an example of a configuration of a vehicle 1 according to a first embodiment. The vehicle 1 is an example of a moving body on which a biological information detection device is mounted. The biological information detection device according to the present embodiment estimates a state of an occupant including a driver 2 driving the vehicle 1 and an occupant other than the driver 2. In the example of FIG. 1, the state of the driver 2 is detected.

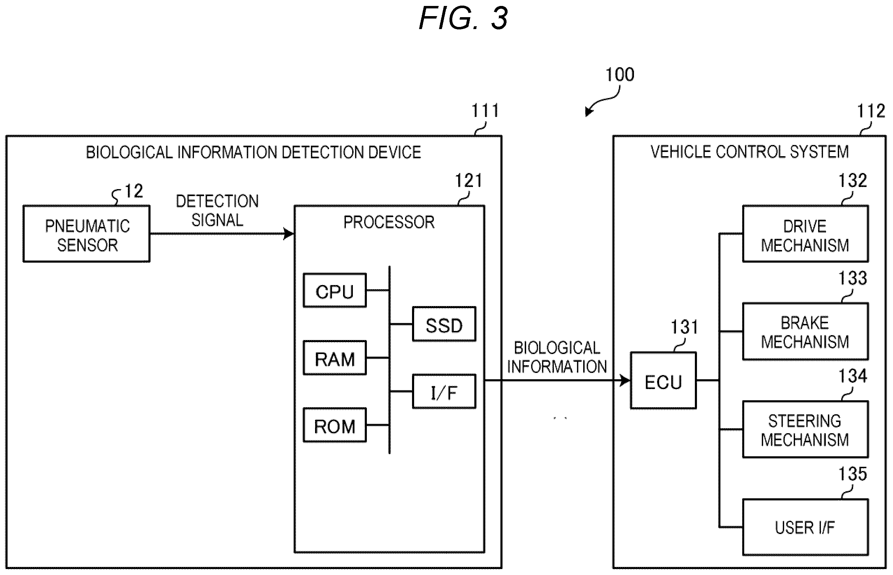

The vehicle 1 according to the present embodiment includes a pneumatic sensor 12 and a camera 14. In addition, the vehicle 1 includes a biological information detection system 100 (see FIG. 3) described later.

The pneumatic sensor 12 is a sensor for detecting a body motion, which is a movement of a body surface of an occupant such as the driver 2. The pneumatic sensor 12 outputs a signal of the detected body motion. In the present embodiment, the pneumatic sensor 12 detects a signal related to a heartbeat (pulsation) of the occupant as the body motion. A signal detected by the pneumatic sensor 12 is referred to as a detection signal.

The pneumatic sensor 12 is installed inside a backrest portion 22. In the present embodiment, a configuration in which the pneumatic sensor 12 is provided as the sensor that detects the heartbeat as the body motion is exemplified, and the sensor that acquires the heartbeat is not limited thereto. For example, a Doppler sensor or the like can be used as a sensor that acquires a heartbeat.

The camera 14 is a device that acquires imaging data obtained by imaging the driver 2 seated on a seat 21. The camera 14 illustrated here is disposed near a boundary between a roof 31 and a windshield 32, and acquires image data including a face of the driver 2 from diagonally above and in front of the driver 2. By analyzing the image data, it is possible to acquire information on a change in appearance such as a movement of a line of sight, an eye movement, and a body motion of the driver 2.

Next, the pneumatic sensor 12 will be described in detail.

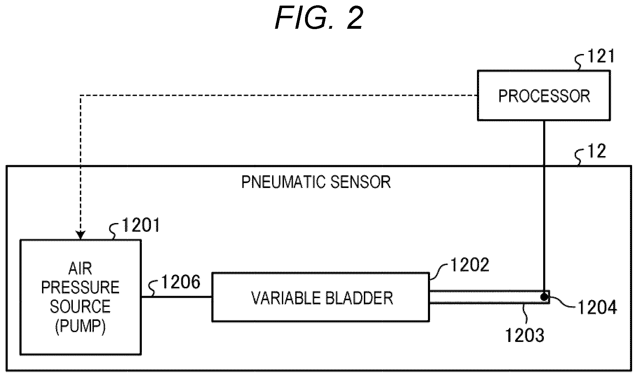

FIG. 2 is a diagram showing an example of a configuration of the pneumatic sensor 12 according to the first embodiment.

The pneumatic sensor 12 includes an air pressure source 1201 constituted by a pump or the like, a variable bladder 1202, a pressure introduction portion 1203, and a pressure sensor 1204 provided at a distal end of the pressure introduction portion 1203.

The air pressure source 1201 and the pressure sensor 1204 are connected to a processor 121 of a biological information detection device 111 (see FIG. 3) described later. The processor 121 detects a body motion as biological information based on the detection signal of the pressure sensor 1204, and individually controls the variable bladder 1202 via the air pressure source 1201.

The variable bladder 1202 is an air bag that is deformable according to a pressure fluctuation caused by a supply pressure and a body motion of the occupant such as the driver 2.

The pressure introduction portion 1203 is connected to one end of the variable bladder 1202, and the other end is provided with the pressure sensor 1204 and closed. The pressure introduction portion 1203 is a tubular member capable of transmitting a supply pressure and a pressure fluctuation caused by a body motion of an occupant such as the driver 2. An inner volume of the pressure introduction portion 1203 is sufficiently smaller than an inner volume of the variable bladder 1202 in order to transmit the pressure change quickly and with high accuracy.

The pressure introduction portion 1203 is made of a material capable of transmitting a pressure to the pressure sensor 1204 while maintaining a shape so as not to absorb the pressure fluctuation even when the variable bladder 1202 is deformed based on the pressure change of air supplied from the air pressure source 1201. Further, the pressure introduction portion 1203 is provided in a predetermined space in a seat of the vehicle 1, is supported by a support member as appropriate, and is implemented so as not to hinder deformation when the supply pressure fluctuates.

The pressure sensor 1204 is provided at the other end of the pressure introduction portion 1203, detects the supply pressure and the pressure caused by the body motion of the occupant, and outputs a pressure detection signal.

In the present embodiment, the air pressure source 1201, the variable bladder 1202, the pressure introduction portion 1203, and the pressure sensor 1204 are stored as the pneumatic sensor 12 in the seat of the vehicle 1.

In the above configuration, the pressure introduction portion 1203 is provided with an air introduction pipe 1206 of the air pressure source 1201 at a position (ideally, a facing position) separated from the position where the variable bladder 1202 is provided so that the pressure introduction portion 1203 is not affected by the pulsation when the supply pressure fluctuates.

Next, the biological information detection system 100 according to the present embodiment will be described.

FIG. 3 is a diagram showing an example of a system configuration and a hardware configuration of the biological information detection system 100 according to the first embodiment. As shown in FIG. 3, the biological information detection system 100 includes a biological information detection device 111 and a vehicle control system 112. Here, the biological information detection device 111 and the vehicle control system 112 are connected by wire or wirelessly.

The biological information detection device 111 is a device that detects biological information of an occupant such as the driver 2 of the vehicle 1. As shown in FIG. 3, the biological information detection device 111 includes the pneumatic sensor 12, the processor 121, and the like.

The processor 121 is an information processing device that performs various kinds of calculation processing according to a program, and is implemented using, for example, a central processing unit (CPU), a random access memory (RAM), a read only memory (ROM), a solid state drive (SSD), and an interface (I/F). The processor 121 loads a program stored in the ROM or the SSD into the RAM and executes calculation processing and control processing for estimating a state of the driver 2. The processor 121 transmits and receives various kinds of information to and from other devices via the I/F.

The processor 121 according to the present embodiment executes processing for detecting the biological information of the occupant such as the driver 2 based on a heartbeat-related detection signal as the detection signal related to the body motion acquired by the pneumatic sensor 12. At this time, the biological information of the occupant such as the driver 2 detected by the processor 121 is output to the vehicle control system 112.

As shown in FIG. 3, the vehicle control system 112 includes an electronic control unit (ECU) 131, a drive mechanism 132, a brake mechanism 133, a steering mechanism 134, a user I/F 135, and the like. The drive mechanism 132 is a mechanism including a drive source (for example, an engine or a motor) of the vehicle 1. The brake mechanism 133 is a mechanism that decelerates and stops the vehicle 1. The steering mechanism 134 is a mechanism that changes a traveling direction of the vehicle 1. The user I/F 135 is a display, a speaker, an operation unit, or the like provided in the vehicle. The ECU 131 is an information processing device that executes various kinds of processing for controlling the drive mechanism 132, the brake mechanism 133, the steering mechanism 134, the user I/F 135, and the like. The ECU 131 according to the present embodiment executes predetermined control using biological information or the like output from the biological information detection device 111. The ECU 131 controls, for example, the drive mechanism 132, the brake mechanism 133, the steering mechanism 134, and the user I/F 135 so that a danger avoidance action is implemented based on the biological information and the like. The danger avoidance action may be, for example, a warning to the driver 2, and deceleration and stop of the vehicle 1.

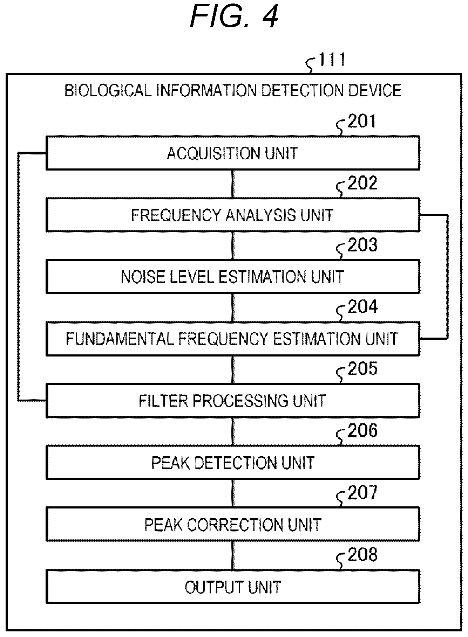

FIG. 4 is a diagram showing an example of a functional configuration of the biological information detection device 111 according to the first embodiment. As shown in FIG. 4, the biological information detection device 111 according to the present embodiment mainly includes an acquisition unit 201, a frequency analysis unit 202, a noise level estimation unit 203, a fundamental frequency estimation unit 204, a filter processing unit 205, a peak detection unit 206, a peak correction unit 207, and an output unit 208. These functional units are implemented by cooperation of hardware elements and software elements (programs and the like) of the biological information detection device 111. At least one of these functional units may include dedicated hardware (such as a circuit).

The acquisition unit 201 acquires a body motion detection signal output from the pneumatic sensor 12, that is, a heartbeat-related detection signal.

The frequency analysis unit 202 performs frequency analysis processing, such as fast Fourier transform, on the detection signal acquired by the acquisition unit 201, and outputs a frequency domain signal of the detection signal as a result of the frequency analysis.

The noise level estimation unit 203 estimates a noise level of the detection signal using the result of the frequency analysis.

The fundamental frequency estimation unit 204 estimates a fundamental frequency of a heartbeat. The fundamental frequency of the heartbeat is roughly a reciprocal of an average heartbeat interval, and is a frequency of a peak appearing around 1 Hz.

The fundamental frequency estimation unit 204 determines whether a signal-to-noise ratio (hereinafter, referred to as an "SN ratio") of the detection signal in an assumed heartbeat fundamental frequency band is less than a second threshold.

Here, the assumed heartbeat fundamental frequency band is a band in which the fundamental frequency is assumed to be present.

When the SN ratio of the detection signal in the assumed fundamental frequency band is equal to or greater than the second threshold, the fundamental frequency estimation unit 204 determines that the noise in the assumed fundamental frequency band is small or an intensity of the detection signal is large. Therefore, the fundamental frequency estimation unit 204 detects a peak of the detection signal in the assumed fundamental frequency band, and estimates a frequency of the peak detected in the assumed fundamental frequency band as the fundamental frequency.

When the SN ratio of the detection signal in the assumed heartbeat fundamental frequency band is less than the second threshold, the fundamental frequency estimation unit 204 determines that the noise in the assumed fundamental frequency band is large or the intensity of the detection signal is low. Therefore, the fundamental frequency estimation unit 204 detects a peak of the frequency domain signal of the detection signal in the assumed heartbeat harmonic band, and estimates the fundamental frequency based on the frequency of the peak detected in the assumed heartbeat harmonic band.

Here, the assumed heartbeat harmonic band is a band in which a frequency higher than the fundamental frequency is assumed to be present. In the present embodiment, the fundamental frequency estimation unit 204 detects a peak of the frequency domain signal of the detection signal in a band that is an integral multiple of the assumed heartbeat fundamental frequency band as the assumed heartbeat harmonic band, and estimates the fundamental frequency of the heartbeat by performing reverse calculation based on a difference in frequency of the peaks detected in the assumed heartbeat harmonic band.

The filter processing unit 205 generates a filter based on the result of the frequency analysis by the frequency analysis unit 202, and applies a generated filter to the detection signal. Here, the filter processing unit 205 generates a bandpass filter as a filter, a passband of which is the fundamental frequency band and a band of predetermined range in a harmonic band.

Here, the fundamental frequency band is a band having a predetermined width based on the fundamental frequency estimated by the fundamental frequency estimation unit 204. The harmonic band is a band of a frequency higher than the fundamental frequency band.

Specifically, the filter processing unit 205 obtains the SN ratio of the detection signal for each of the fundamental frequency band and the harmonic band, and generates a bandpass filter in which only a band in which the SN ratio is equal to or greater than a predetermined first threshold is set as a passband.

The peak detection unit 206 detects biological information, which is a heartbeat interval, based on a detection signal before the application of the bandpass filter (hereinafter, referred to as "before filter application") corresponding to the peak of the detection signal after the application of the bandpass filter (hereinafter, referred to as "after filter application"). That is, the peak of the detection signal before filter application is obtained for each peak of the detection signal after filter application. Then, the peak detection unit 206 detects an array of the peaks of the detection signal based on the peaks of the detection signal after the application and the peaks of the detection signal before filter application.

Specifically, for each of the peaks (first peaks) in the detection signal after filter application, the peak detection unit 206 detects the array of the peaks of the detection signal as the biological information according to positions of a peak (a second peak) immediately before the time of the first peak in the peaks of the detection signal before filter application and a peak (a third peak) immediately after the time of the first peak in the peaks of the detection signal before filter application.

The peak correction unit 207 corrects the position of the peak of the detection signal when the peak of the detection signal is present in a predetermined outlier range.

The output unit 208 outputs the heartbeat interval based on the peak of the corrected detection signal to the vehicle control system 112 as the biological information.

Next, biological information detection processing performed by the biological information detection device 111 according to the present embodiment will be described.

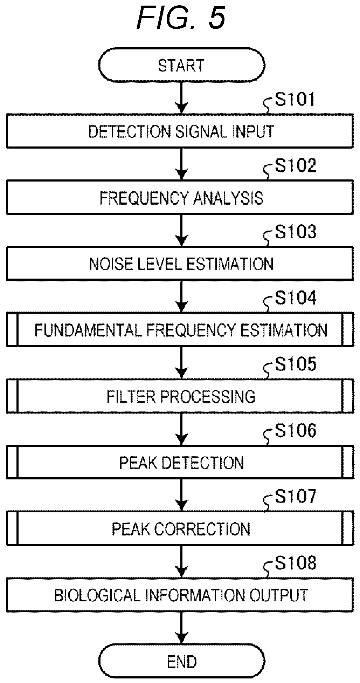

FIG. 5 is a flowchart showing an example of a procedure of the biological information detection processing according to the first embodiment.

First, the acquisition unit 201 acquires a detection signal from the pneumatic sensor 12 (S101). Next, the frequency analysis unit 202 performs frequency analysis on the acquired detection signal (S102). Accordingly, a frequency domain signal of the detection signal is obtained. Next, the noise level estimation unit 203 estimates a noise level of the detection signal (S103). Specifically, in order to calculate the SN ratio, the noise level estimation unit 203 estimates a noise approximate line obtained by fitting a noise to a curve of the frequency domain signal of the detection signal.

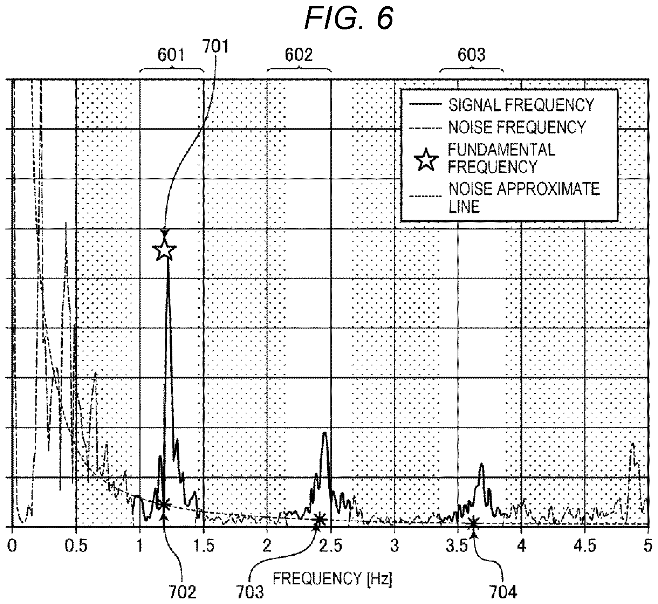

FIG. 6 is a diagram showing an example of a waveform of a frequency domain signal that is a frequency analysis result according to the first embodiment. In FIG. 6, a horizontal axis represents a frequency, and a vertical axis represents an intensity of the detection signal.

Waveforms indicated by a solid line and a one-dot chain line are frequency domain signals of the detection signal. Here, the waveform indicated by the one-dot chain line is a noise waveform. The waveform indicated by the solid line is the waveform of the detection signal. In FIG. 6, a dotted line is a noise approximate line indicating a noise level estimated by the noise level estimation unit 203.

Returning to FIG. 5, next, the fundamental frequency estimation unit 204 executes fundamental frequency estimation processing of estimating the fundamental frequency of the heartbeat (S104).

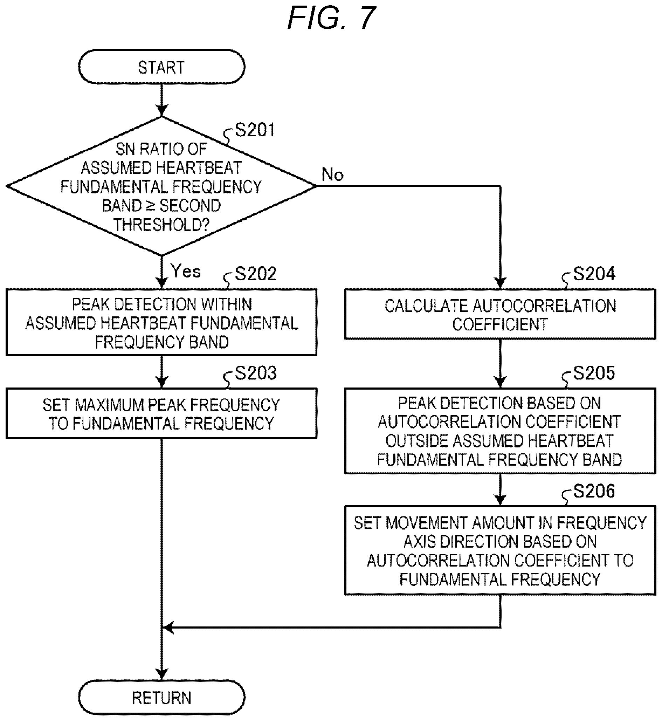

FIG. 7 is a flowchart showing an example of a procedure of the fundamental frequency estimation processing according to the first embodiment.

First, the fundamental frequency estimation unit 204 obtains the SN ratio of the detection signal in the assumed heartbeat fundamental frequency band, and determines whether the SN ratio is equal to or greater than the second threshold (S201). Here, the fundamental frequency estimation unit 204 calculates the SN ratio based on a signal intensity (that is, an amplitude) in the assumed heartbeat fundamental frequency band with a portion denoted by the reference numeral 702 of a noise approximate line shown in FIG. 6 as the noise level.

When the SN ratio of the detection signal in the assumed heartbeat fundamental frequency band is equal to or greater than the second threshold (S201: Yes), the signal intensity with respect to the noise is sufficient, and the fundamental frequency estimation unit 204 determines that the fundamental frequency can be directly estimated based on the assumed heartbeat fundamental frequency band. Therefore, the fundamental frequency estimation unit 204 detects the peak of the frequency domain signal of the detection signal in the assumed heartbeat fundamental frequency band (S202).

Then, the fundamental frequency estimation unit 204 sets a frequency of a maximum peak among the peaks detected in the assumed heartbeat fundamental frequency band as the fundamental frequency (S203). In the example of FIG. 6, the fundamental frequency estimation unit 204 sets the maximum peak indicated by a star mark as the fundamental frequency.

On the other hand, when the SN ratio of the detection signal of the assumed heartbeat fundamental frequency band is less than the second threshold in S201 (S201: No), the signal intensity with respect to the noise is insufficient, and it is difficult for the fundamental frequency estimation unit 204 to directly estimate the fundamental frequency based on the assumed heartbeat fundamental frequency band. For example, the noise level is high. Therefore, the fundamental frequency estimation unit 204 does not directly detect the fundamental frequency based on the assumed heartbeat fundamental frequency band, but estimates the fundamental frequency based on the peak of the frequency domain signal of the detection signal in the assumed heartbeat fundamental frequency band.

In the present embodiment, the fundamental frequency estimation unit 204 detects a peak of the frequency domain signal of the detection signal in a band that is an integral multiple of the assumed heartbeat fundamental frequency band as the assumed heartbeat harmonic band. Specifically, the fundamental frequency estimation unit 204 calculates an autocorrelation coefficient by multiplying a signal intensity by a window function that is amplified toward a higher frequency side (S204).

Then, the fundamental frequency estimation unit 204 detects a peak based on the autocorrelation coefficient in a band outside the assumed fundamental frequency band (S205). Specifically, the fundamental frequency estimation unit 204 calculates the autocorrelation coefficient with a signal before the movement while moving the frequency domain signal of the detection signal to a high frequency side along a frequency axis, and determines that a state in which the autocorrelation coefficient has a maximum value is a state in which the peaks of the signals before and after the movement overlap. Then, the fundamental frequency estimation unit 204 sets a movement amount in a frequency axis direction determined based on the autocorrelation coefficient as the fundamental frequency (S206).

For example, the fundamental frequency estimation unit 204 calculates the autocorrelation coefficient while shifting the signal toward an assumed heartbeat harmonic band that is three times the assumed heartbeat fundamental frequency band with respect to an assumed heartbeat harmonic band that is twice the assumed heartbeat fundamental frequency band. Then, the fundamental frequency estimation unit 204 determines that a portion where the autocorrelation coefficient is the maximum is a portion that matches the peak in the three times assumed heartbeat harmonic band. A slide width (for example, the bandwidth of 1.2 Hz) at this time is a width between the peak of the twice assumed heartbeat harmonic band and the peak of the three times assumed heartbeat harmonic band. Therefore, the fundamental frequency estimation unit 204 estimates a frequency corresponding to the width as the fundamental frequency of the heartbeat based on the peak of the twice assumed heartbeat harmonic band.

As described above, when the fundamental frequency of the heartbeat is estimated, the fundamental frequency estimation unit 204 sets a band of a predetermined width as the fundamental frequency band based on the fundamental frequency, and sets a band of a predetermined width on a harmonic side that is an integral multiple of a fundamental frequency band 601 as the harmonic band. In the example of FIG. 6, the fundamental frequency band 601, a harmonic band 602 twice the fundamental frequency band 601, and a harmonic band 603 three times the fundamental frequency band 601 are set.

Then, the processing returns to a caller.

Returning to FIG. 5, when the fundamental frequency estimation processing (S104) ends, the filter processing unit 205 executes filter processing of generating a bandpass filter and applying the bandpass filter to the detection signal (S105).

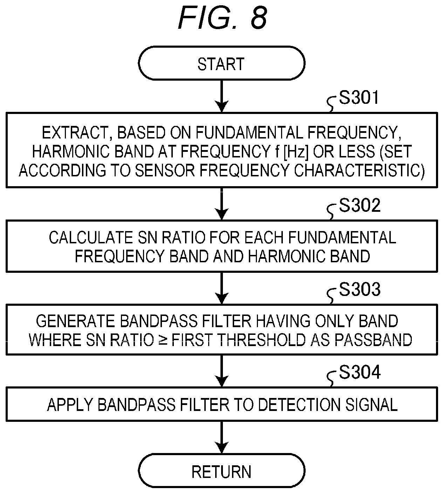

FIG. 8 is a flowchart showing an example of a procedure of filter processing according to the first embodiment.

First, the filter processing unit 205 extracts, based on the fundamental frequency, a harmonic band equal to or lower than a frequency f corresponding to a frequency response characteristic of the pneumatic sensor 12 (S301). Harmonic responsiveness varies depending on the frequency response characteristic of the sensor. Therefore, in the case of the pneumatic sensor 12 with high responsiveness, the filter processing unit 205 extracts a harmonic band, for example, below a band five times the fundamental frequency, and in the case of the pneumatic sensor 12 with low responsiveness, the filter processing unit 205 extracts a harmonic band, for example, below a band three times the fundamental frequency.

Next, the filter processing unit 205 calculates the SN ratio of the detection signal for each of the fundamental frequency band and the harmonic band (S302). In the example of FIG. 6, the filter processing unit 205 calculates the SN ratio using a noise level at the reference numeral 702 on the noise approximate line in the fundamental frequency band, a noise level at the reference numeral 703 on the noise approximate line in the harmonic band 602 that is twice the fundamental frequency band, and a noise level at the reference numeral 704 on the noise approximate line in the harmonic band 603 that is three times the fundamental frequency band.

Next, the filter processing unit 205 generates a bandpass filter in which only a band in which the SN ratio is equal to or greater than the first threshold is set as a passband from among the fundamental frequency band and the harmonic band (S303). In the example of FIG. 6, the filter processing unit 205 extracts, as the harmonic bands, the band 602 that is twice the fundamental frequency band 601 and the band 603 that is three times the fundamental frequency band 601, and does not extract a band (around 5 Hz) that is four times the fundamental frequency band 601.

Then, the filter processing unit 205 applies the generated bandpass filter to the detection signal. Accordingly, the detection signal of only the passband is extracted. Then, the processing returns to the caller.

Returning to FIG. 5, when the filter processing (S105) is completed, the peak detection unit 206 executes peak detection processing of detecting a peak of the detection signal (S106).

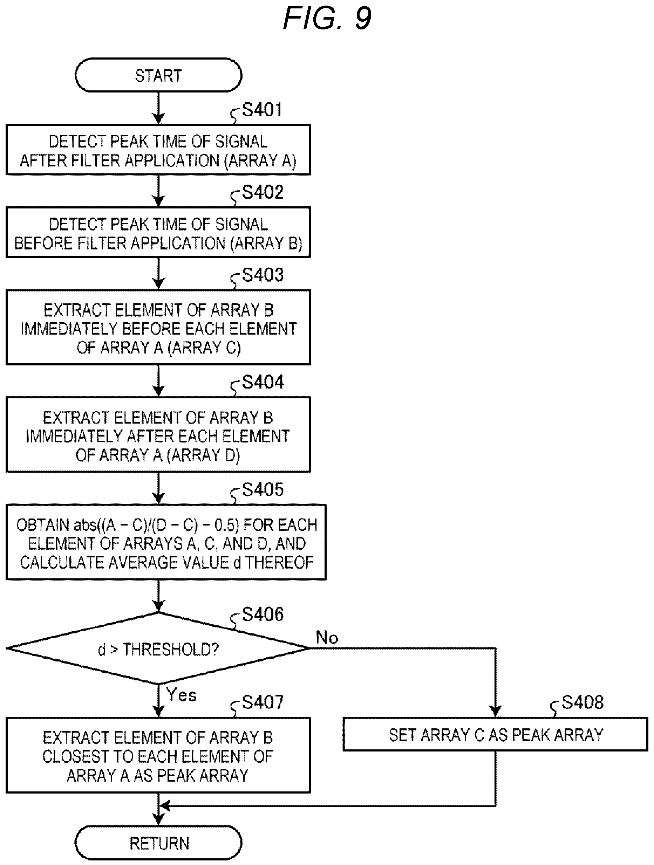

FIG. 9 is a flowchart showing an example of a procedure of peak detection processing according to the first embodiment.

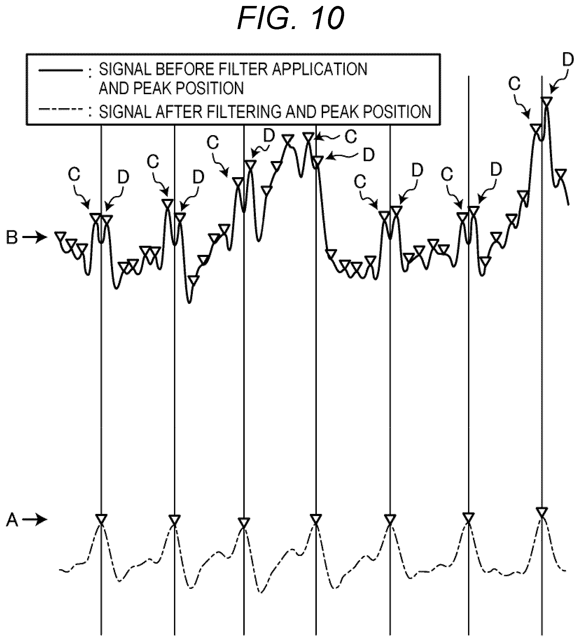

FIG. 10 is a diagram showing a comparison between a detection signal before filter application and a detection signal after filter application according to the first embodiment. In FIG. 10, a horizontal axis represents a time, and a vertical axis represents an intensity. In FIG. 10, an upper side solid line graph indicates a detection signal and a peak position before filter application. A lower side one-dot chain line graph indicates a detection signal and a peak position after filter application. In each waveform, the peak position is indicated by an inverted triangular mark.

First, the peak detection unit 206 detects a peak time of the detection signal after filter application (S401). As shown in FIG. 10, an array of peaks (first peaks) of the detected detection signal after filter application is defined as an array A.

Next, the peak detection unit 206 detects a peak time of the detection signal before filter application (S402). As shown in FIG. 10, an array of peaks of the detected detection signal before filter application is defined as an array B.

Next, the peak detection unit 206 extracts a peak (second peak) which is an element of the array B immediately before the time of each element of the array A of the peak of the detection signal after filter application (S403). As shown in FIG. 10, the extracted elements of the array B are denoted by C.

Next, the peak detection unit 206 extracts a peak (third peak) which is an element of the array B immediately after the time of each element of the array A of the peak of the detection signal after filter application (S404). As shown in FIG. 10, the extracted elements of the array B are denoted by D.

Next, the peak detection unit 206 obtains a value X for each element of the arrays A, C, and D by the following formula (1), and further calculates an average value d of X calculated for each element (S405).

$$X = \mathrm{abs}((A - C)/(D - C) - 0.5) \quad (1)$$

Here, X is a value indicating a degree of deviation from the center of the peak time immediately before and after the peak time after filter application (in the detection signal before filter application).

That is, in each of the peaks (first peaks) in the detection signal after filter application, when a position of the first peak is separated from a central position of an interval between the second peak and the third peak by a predetermined distance or greater, the peak detection unit 206 sets either one of the second peak and the third peak that is closer closest to the first peak as a peak of the detection signal and detects the array of the peaks of the detection signal as the biological information.

When the position of the first peak is within the predetermined distance from the central position of the interval between the second peak and the third peak, the peak detection unit 206 detects the array of the peaks of the detection signal with the second peak as the peak of the detection signal.

Specifically, the following processing is performed.

The peak detection unit 206 determines whether the average value d of X is greater than the first threshold as the predetermined distance, that is, whether the second peak and the third peak are separated from the center (S406). When the average value d is greater than the first threshold (S406: Yes), it means that the second peak and the third peak are separated from the center. Therefore, the peak detection unit 206 extracts the element of the array B (that is, the peak of the detection signal after filter application) closest to the element of the array A (that is, each peak time of the detection signal after filter application), and detects the extracted element as the peak array of the detection signal (S407).

On the other hand, in S406, when the average value d is smaller than the first threshold (S406: No), it means that the second peak and the third peak are not so far away from the center. Therefore, the peak detection unit 206 detects the array C (that is, the element of the array B immediately before the position of the detection signal after filter application corresponding to each element of the array A) as the peak array of the detection signal (S408).

In this way, the peak detection unit 206 detects the peak array of the detection signal. Then, the processing returns to the caller.

Returning to FIG. 5, when the peak detection processing (S106) is completed, the peak correction unit 207 executes peak correction processing of correcting the peak of the detection signal detected in S106 (S107).

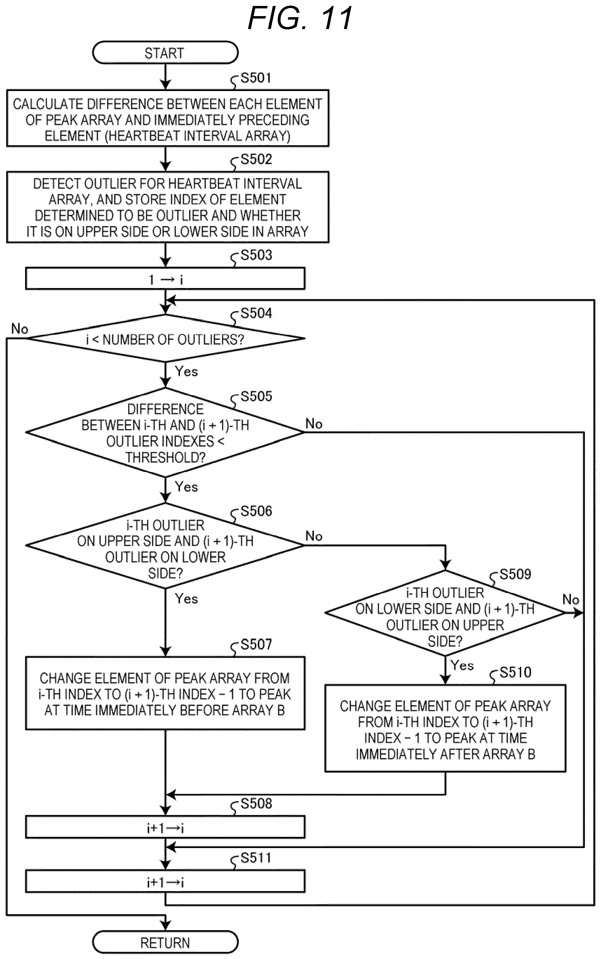

FIG. 11 is a flowchart showing an example of a procedure of the peak correction processing according to the first embodiment.

First, the peak correction unit 207 calculates a difference (that is, an interval) between each element of the array of the peaks of the detection signal and the immediately preceding element (S501). The interval between the elements for the elements is referred to as a heartbeat interval array.

Next, the peak correction unit 207 detects whether there is an outlier in the heartbeat interval array obtained in S501, and stores in the array an index of the element determined to be the outlier and whether the index element determined to be the outlier deviates to an upper side or a lower side (S502).

Here, an upper side outlier corresponds to, for example, a case of exceeding an average value + standard deviation * n (n is an integer). A lower side outlier corresponds to, for example, a value less than an average value - standard deviation * n (n is an integer). However, these are merely examples, and the embodiment disclosed here is not limited thereto.

Next, the peak correction unit 207 initializes a counter i to 1 (S503).

Next, it is determined whether a value of the counter i is less than the number of outliers detected in S502 (S504).

When the value of the counter i is less than the number of outliers (S504: Yes), the peak correction unit 207 determines whether a difference between the i-th and (i + 1)-th indexes of the element serving as the outlier is less than a predetermined threshold (S505). When the difference between the i-th and (i + 1)-th indexes of the element serving as the outlier is equal to or greater than the predetermined threshold (S505: No), the peak correction unit 207 increments the counter i (S511), the processing returns to S504, and the processing is repeatedly executed from S504.

On the other hand, in S505, when the difference between the i-th and (i + 1)-th indexes of the element serving as the outlier is less than the predetermined threshold (S505: Yes), the peak correction unit 207 determines whether the i-th index element is an upper side outlier and the (i + 1)-th index element is a lower side outlier (S506).

When the i-th index element is the upper side outlier and the (i + 1)-th index element is the lower side outlier (S506: Yes), it means that the interval between the i-th index element and the (i + 1)-th index element is narrowed. Therefore, the peak correction unit 207 changes the elements of the peak array of the detection signal from the i-th index element to the (i + 1)-th index - 1 element to a peak at a time immediately before the array B (a peak array before filter application) (S507). Then, the peak correction unit 207 increments the counter i (S508). Next, the peak correction unit 207 further increments the counter i (S511), the processing returns to S504, and the processing is repeatedly executed from S504.

When the i-th index element is not the upper side outlier or the (i + 1)-th index element is not the lower side outlier in S506 (S506: No), the peak correction unit 207 determines whether the i-th outlier is on the lower side and the (i + 1)-th outlier is on the upper side (S509).

When the i-th outlier is not on the lower side or the (i + 1)-th outlier is not on the upper side (S509: No), the peak correction unit 207 increments the counter i (S511), the processing returns to S504, and the processing is repeatedly executed from S504.

When the i-th outlier is on the lower side and the (i + 1)-th outlier is on the upper side in S509 (S509: Yes), it means that the interval between the i-th index element and the (i + 1)-th index element is widened. Therefore, the peak correction unit 207 changes the elements of the peak array of the detection signal from the i-th index element to the (i + 1)-th index - 1 element to a peak at a time immediately after the array B (a peak array before filter application) (S510). Then, the peak correction unit 207 increments the counter i (S508). Next, the peak correction unit 207 further increments the counter i (S511), the processing returns to S504, and the processing is repeatedly executed from S504.

In S504, when the value of the counter i is equal to or greater than the number of outliers (S504: No), the processing returns to the caller.

Returning to FIG. 5, when the peak correction processing (S107) ends, the output unit 208 outputs the heartbeat interval based on the peak of the detection signal to the vehicle control system 112 as the biological information (S108).

As described above, the biological information detection device 111 according to the present embodiment includes the pneumatic sensor 12 that detects information on a body motion of a person, the frequency analysis unit 202 that performs frequency analysis on a detection signal of the information on the body motion by the pneumatic sensor 12, the filter processing unit 205 that generates a filter based on a result of the frequency analysis and applies the generated filter to the detection signal, and the peak detection unit 206 that detects biological information that is a heartbeat interval based on a detection signal before applying the filter corresponding to a peak of a detection signal after applying the filter.

Therefore, according to the present embodiment, the biological information that is a heartbeat interval is detected using raw data, which is a detection signal before the filter processing corresponding to a peak of a signal after the filter processing, instead of a signal after the filter processing of a detection signal of information on the body motion, and thus it is possible to detect the biological information with high accuracy. Therefore, according to the present embodiment, since only the pneumatic sensor 12 is used when the detection signal is detected, a device configuration is simplified and manufacturing cost of the device can be reduced as compared with the case in which a plurality of sensors are used.

The biological information detection device 111 according to the present embodiment further includes the fundamental frequency estimation unit 204 that estimates the fundamental frequency of the body motion, and the filter processing unit 205 generates a filter having, as a passband, a fundamental frequency band that is a band based on the estimated fundamental frequency and a band of a predetermined range in a harmonic band that is a band of a frequency higher than the fundamental frequency band, and applies the generated filter to the detection signal.

Therefore, according to the present embodiment, by applying the filter, a frequency related to the detection signal can be easily extracted, and the frequency adjusted by the filter can be extracted, so that the biological information can be detected with higher accuracy.

In addition, in the biological information detection device 111 according to the present embodiment, the filter processing unit 205 obtains an SN ratio of the detection signal for each of the fundamental frequency band and the harmonic band, and generates the filter having only the band in which the SN ratio is equal to or greater than the first threshold as the passband.

Therefore, according to the present embodiment, since the filter in which only the band having a high SN ratio is set as the passband is applied to the detection signal in the fundamental frequency band and the harmonic band, it is possible to reduce a waveform deterioration in the detection signal and to detect the biological information with higher accuracy.

In addition, in the biological information detection device 111 according to the present embodiment, the filter processing unit 205 generates the filter having, as the passband, the fundamental frequency band and a band corresponding to a frequency response characteristic of the pneumatic sensor 12 among the harmonic band.

Therefore, according to the present embodiment, accuracy of the detection signal after filter application is improved. Accordingly, the biological information can be detected with higher accuracy.

The biological information detection device 111 according to the present embodiment includes the fundamental frequency estimation unit 204 that, when an SN ratio of the detection signal in an assumed heartbeat fundamental frequency band, which is a band in which the fundamental frequency is assumed to be present, is less than a second threshold, detects a peak of a frequency domain signal of the detection signal in an assumed heartbeat harmonic band, which is a band in which a frequency higher than the fundamental frequency is assumed to be present, and estimates the fundamental frequency based on a frequency of the peak detected in the assumed heartbeat harmonic band.

Therefore, according to the present embodiment, even when the SN ratio of the detection signal is low due to noise or the like in the assumed heartbeat fundamental frequency band, the peak is unclear, and it is difficult to estimate the fundamental frequency, the fundamental frequency can be estimated based on the peak detected in the assumed heartbeat harmonic band. Therefore, according to the present embodiment, even when the SN ratio of the detection signal is low in the assumed heartbeat fundamental frequency band, the fundamental frequency can be accurately estimated, and as a result, the biological information can be detected with higher accuracy.

In the biological information detection device 111 according to the present embodiment, the fundamental frequency estimation unit 204 detects, as the assumed heartbeat harmonic band, a peak of the frequency domain signal of the detection signal in a band that is an integral multiple of the assumed heartbeat fundamental frequency band, and estimates the fundamental frequency by performing reverse calculation based on a difference in frequency of the peaks detected in the assumed heartbeat harmonic band.

Therefore, according to the present embodiment, even when the SN ratio of the detection signal is low due to noise or the like in the assumed heartbeat fundamental frequency band, the peak is unclear, and it is difficult to estimate the fundamental frequency, the fundamental frequency is estimated by performing the reverse calculation based on a difference in frequency of the peaks detected in the assumed heartbeat harmonic band that is an integral multiple of the assumed heartbeat fundamental frequency band, and thus it is possible to improve robustness and to accurately and easily estimate the fundamental frequency. Therefore, according to the present embodiment, even when the SN ratio of the detection signal is low in the assumed heartbeat fundamental frequency band, the fundamental frequency can be accurately and easily estimated, and as a result, the biological information can be detected with higher accuracy.

In the biological information detection device 111 according to the present embodiment, with respect to each of first peaks that are peaks in the detection signal after the application of the filter, the peak detection unit 206 detects an array of peaks of the detection signal as the biological information according to positions of a second peak that is a peak immediately before a time of the first peak in the detection signal before the application of the filter and a third peak that is a peak immediately after the time of the first peak in the detection signal before the application of the filter.

Therefore, according to the present embodiment, the array of the peaks of the detection signal is detected as the biological information by using the second peak immediately before the time of the first peak and the third peak immediately after the time of the first peak with respect to the first peak in the detection signal after filter application, and thus it is possible to detect the biological information which is the heartbeat interval with higher accuracy.

In addition, in the biological information detection device 111 according to the present embodiment, in each of the first peaks, when a position of the first peak is separated from a central position of an interval between the second peak and the third peak by a predetermined distance or greater, the peak detection unit 206 sets one of the second peak and the third peak that is closer to the first peak as a peak of the detection signal, and detects the array of the peaks of the detection signal as the biological information.

Therefore, according to the present embodiment, when the position of the first peak is separated from the central position of the interval between the second peak and the third peak by a predetermined distance or greater, one of the second peak and the third peak that is closer to the first peak is set as a peak of the detection signal, so that the biological information which is the heartbeat interval can be detected with higher accuracy.

In the biological information detection device 111 according to the present embodiment, when the position of the first peak is within the predetermined distance from the central position of the interval between the second peak and the third peak, the peak detection unit 206 further detects the second peak as the peak of the detection signal and detects the array of the peaks of the detection signal as the biological information. Therefore, according to the present embodiment, when the position of the first peak is within the predetermined distance from the central position of the interval between the second peak and the third peak, the second peak is set as the peak of the detection signal, so that the biological information which is the heartbeat interval can be detected with higher accuracy.

The biological information detection device 111 according to the present embodiment further includes the peak correction unit 207 that corrects a position of a peak of the detection signal when the peak of the detection signal is present in a predetermined outlier range. Therefore, according to the present embodiment, even when there is an error in the peak of the detection signal detected by the peak detection unit 206, it is possible to detect the biological information which is the heartbeat interval with higher accuracy by the correction.

In the biological information detection device 111 according to the present embodiment, the pneumatic sensor 12 detects information on a heartbeat of a person as the information on the body motion of the person. Therefore, according to the present embodiment, since a signal related to the heartbeat is detected as the body motion and various kinds of processing are performed, it is possible to detect the biological information which is the heartbeat interval with higher accuracy.

In the biological information detection device 111 according to the present embodiment, the pneumatic sensor 12 detects the information on the heartbeat by a pneumatic change of the air bag capable of pressing against the person while the person is seated. Therefore, according to the present embodiment, since the information on the heartbeat is detected using an existing component, it is possible to simplify a device structure and reduce manufacturing cost of the device.

Modification

In the above embodiment, the peak detection unit 206 detects the array of the peaks of the detection signal by using all the peaks of the detection signal after filter application, and the present disclosure is not limited thereto. For example, the peak detection unit 206 can be implemented to detect the array of the peaks of the detection signal while ignoring a peak deviated from the fundamental frequency among the peaks of the detection signal after filter application.

In this case, by ignoring the peak deviated from the fundamental frequency, the biological information which is the heartbeat interval can be detected with higher accuracy.

Second Embodiment

In the first embodiment and the modification, a body motion of a person is detected and a heartbeat interval based on a peak of a detection signal is detected as biological information, whereas respiration of a person is not considered. In a second embodiment, a heartbeat interval based on a peak of a detection signal is detected as the biological information in consideration of the respiration of the person.

The configuration of the vehicle 1, the configuration of the biological information detection system 100, and the configuration of the biological information detection device 111 according to the second embodiment are the same as those of the first embodiment.

The fundamental frequency estimation unit 204 of the biological information detection device 111 according to the present embodiment has functions same as those of the first embodiment, detects a body motion due to respiration based on a frequency domain signal of a detection signal as respiration body motion information, and estimates a fundamental frequency of a heartbeat based on the detected respiration body motion information and a frequency of a peak detected in the assumed heartbeat fundamental frequency band or the assumed heartbeat harmonic band.

Next, biological information detection processing according to the present embodiment will be described.

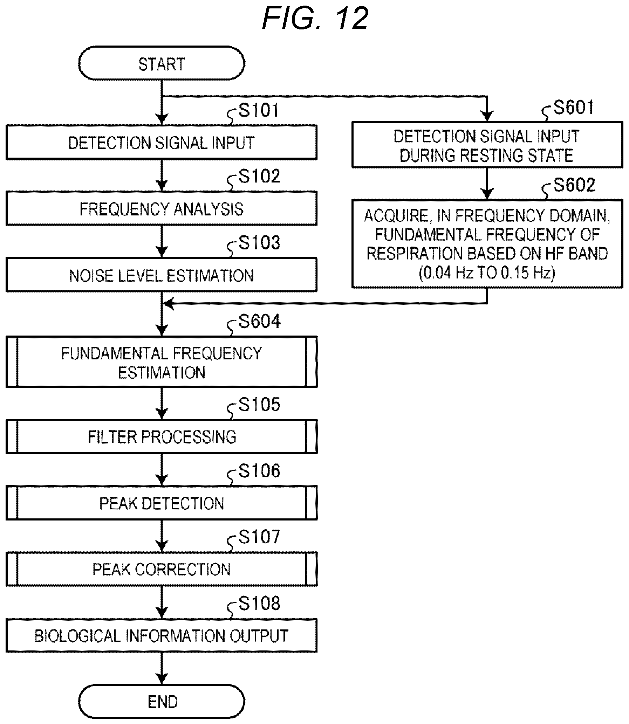

FIG. 12 is a flowchart showing an example of a procedure of the biological information detection processing according to the second embodiment. The processing from an input of the detection signal from the pneumatic sensor 12 to the noise level estimation (S101 to S103) is performed in the same manner as in the first embodiment.

In the present embodiment, the following processing is executed in parallel or in advance.

That is, when an occupant is at rest, the acquisition unit 201 inputs a detection signal from the pneumatic sensor 12 (S601). Next, the fundamental frequency estimation unit 204 acquires a fundamental frequency of respiration from an HF band (0.04 Hz to 0.15 Hz) in a frequency domain (S602).

Next, the fundamental frequency estimation unit 204 executes fundamental frequency estimation processing (S603).

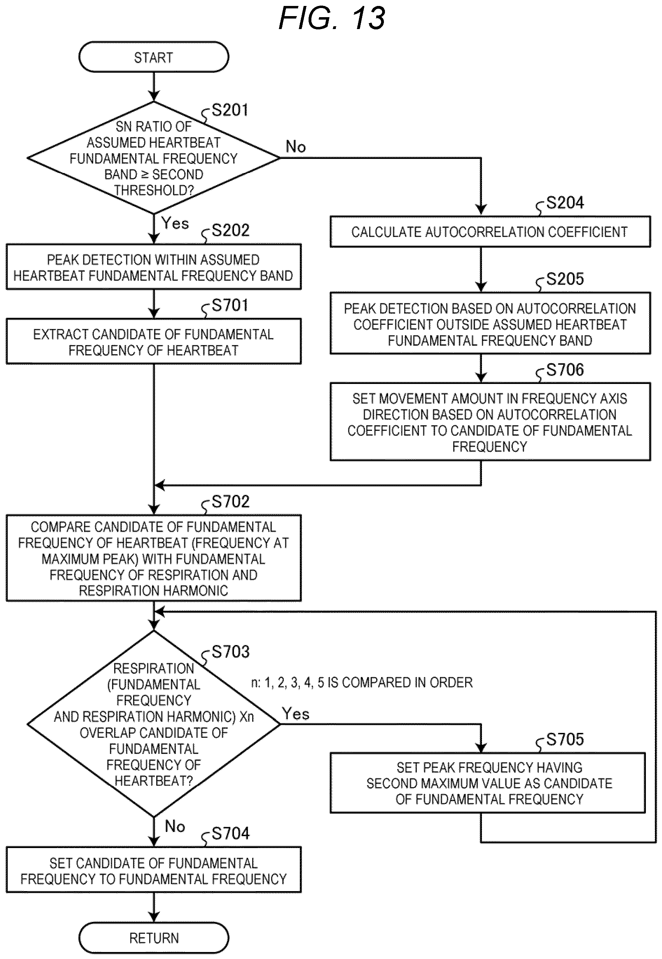

FIG. 13 is a flowchart showing an example of a procedure of the fundamental frequency estimation processing according to the second embodiment.

As in the first embodiment, the fundamental frequency estimation unit 204 determines whether an SN ratio of the detection signal in the assumed heartbeat fundamental frequency band is equal to or greater than the second threshold (S201). When the SN ratio is less than the second threshold (S201: No), the fundamental frequency estimation unit 204 calculates the autocorrelation coefficient (S204) and performs peak detection based on the autocorrelation coefficient outside the assumed heartbeat fundamental frequency band (S205) as in the first embodiment. Then, similarly to the first embodiment, the fundamental frequency estimation unit 204 sets a movement amount in a frequency axis direction determined based on the autocorrelation coefficient as a candidate of the fundamental frequency of the heartbeat (S706). Then, the processing proceeds to S702.

When the SN ratio of the detection signal in the assumed heartbeat fundamental frequency band is equal to or greater than the second threshold in S201 (S201: No), the fundamental frequency estimation unit 204 detects a peak in the assumed heartbeat fundamental frequency band as in the first embodiment (S202). Next, the fundamental frequency estimation unit 204 extracts a frequency having a maximum peak as the candidate of the fundamental frequency of the heartbeat (S701).

Next, in S702, the fundamental frequency estimation unit 204 compares the candidate of the fundamental frequency of the heartbeat with the fundamental frequency of respiration and a respiration harmonic with respect to the fundamental frequency of respiration set in S205 (S702). Specifically, when the fundamental frequency and the respiration harmonic with respect to the fundamental frequency of respiration are denoted by Xn (n is an integer), the fundamental frequency estimation unit 204 determines whether the fundamental frequency of respiration and the respiration harmonic overlap the candidate of the fundamental frequency of the heartbeat (S703). Specifically, the fundamental frequency estimation unit 204 compares each of the fundamental frequency of respiration and the respiration harmonic with the candidate of the fundamental frequency of the heartbeat while increasing n, and determines whether both overlap.

When the fundamental frequency of respiration and the respiration harmonic do not overlap the candidate of the fundamental frequency of the heartbeat (S703: No), the fundamental frequency estimation unit 204 sets the frequency of the detected candidate of the fundamental frequency as the fundamental frequency of the heartbeat (S704). Then, the processing returns to the caller.

On the other hand, in S703, when the fundamental frequency of respiration and the respiration harmonic overlap the candidate of the fundamental frequency of the heartbeat (S703: Yes), the fundamental frequency estimation unit 204 estimates the frequency of a second maximum peak after the maximum peak among the detected candidates of the fundamental frequency as the fundamental frequency of the heartbeat (S705). Then, the processing returns to step S703, and the processing from step S703 is repeatedly executed while increasing n. Accordingly, a frequency of a peak that does not overlap the fundamental frequency of respiration and the respiration harmonic is detected based on the assumed heartbeat fundamental frequency band, and is estimated as the fundamental frequency of the heartbeat.

Returning to FIG. 12, when the fundamental frequency estimation processing (S604) is completed, the filter processing to the biological information output processing (S105 to S108) are executed as in the first embodiment.

As described above, in the biological information detection device 111 according to the present embodiment, the fundamental frequency estimation unit 204 detects the body motion due to respiration based on a frequency domain signal of a detection signal as respiration body motion information, and estimates a fundamental frequency of a heartbeat based on the detected respiration body motion information and a frequency of a peak detected in the assumed heartbeat fundamental frequency band or the assumed heartbeat harmonic band. Therefore, in the present embodiment, since the fundamental frequency of the heartbeat is estimated in consideration of the frequency of respiration, the fundamental frequency can be estimated more accurately. Accordingly, the biological information which is the heartbeat interval can be detected with higher accuracy.

In the biological information detection device 111 according to the present embodiment, the fundamental frequency estimation unit 204 detects a fundamental frequency of respiration as the respiration body motion information, determines whether the detected fundamental frequency of respiration and a respiration harmonic with respect to the fundamental frequency of respiration overlap the frequency of the peak detected in the assumed heartbeat fundamental frequency band, estimates the frequency of the peak detected in the assumed heartbeat fundamental frequency band or the assumed heartbeat harmonic band as the fundamental frequency of the detection signal when the frequencies do not overlap, and detects the frequency of the peak that does not overlap the fundamental frequency of the respiration and the respiration harmonic from the assumed heartbeat fundamental frequency band, and estimates the frequency as the fundamental frequency of the body motion when the frequencies overlap. Therefore, according to the present embodiment, since the fundamental frequency of the heartbeat is estimated after removing the frequency of the respiration, the fundamental frequency of the heartbeat can be estimated more accurately. Accordingly, the biological information which is the heartbeat interval can be detected with higher accuracy.

In the above embodiment, the biological information detection device 111 detects biological information of an occupant such as the driver 2 of the vehicle 1, but a detection target is not limited to the occupant of the vehicle 1, and the biological information detection device 111 may be any device that detects biological information of a person.

The program for causing a computer (the processor 121 and the like) to achieve the functions of the biological information detection device 111 according to the embodiments and modification described above may be provided by being recorded in a computer-readable recording medium such as a CD-ROM, a flexible disk (FD), a CD-R, or a digital versatile disk (DVD) in an installable or executable file format.

The program may be stored on a computer connected to a network such as the Internet and provided by being downloaded via the network. The program may be provided or distributed via a network such as the Internet.

The principles, preferred embodiment and mode of operation of the present invention have been described in the foregoing specification. However, the invention which is intended to be protected is not to be construed as limited to the particular embodiments disclosed. Further, the embodiments described herein are to be regarded as illustrative rather than restrictive. Variations and changes may be made by others, and equivalents employed, without departing from the spirit of the present invention. Accordingly, it is expressly intended that all such variations, changes and equivalents which fall within the spirit and scope of the present invention as defined in the claims, be embraced thereby.

**Claims**

1. A biological information detection device (111) comprising:

   a sensor (12) configured to detect information on a body motion of a person;
   a frequency analysis unit (202) configured to perform frequency analysis on a detection signal of the information on the body motion detected by the sensor;
   a filter processing unit (205) configured to generate a filter based on a result of the frequency analysis and apply the generated filter to the detection signal; and
   a detection unit (206) configured to detect biological information that is a heartbeat interval based on a detection signal before applying the filter corresponding to a peak of a detection signal after applying the filter.

2. The biological information detection device according to claim 1, further comprising:

   an estimation unit (204) configured to estimate a fundamental frequency of the body motion, wherein
   the filter processing unit generates the filter having, as a passband, a fundamental frequency band that is a band based on the estimated fundamental frequency and a band in a predetermined range in a harmonic band that is a band of a frequency higher than the fundamental frequency band, and applies the generated filter to the detection signal.

3. The biological information detection device according to claim 2, wherein
   the filter processing unit obtains a signal-to-noise ratio of the detection signal for each of the fundamental frequency band and the harmonic band, and generates the filter having, as the passband, only a band in which the signal-to-noise ratio is equal to or greater than a first threshold.

4. The biological information detection device according to claim 2, wherein
   the filter processing unit generates the filter having, as the passband, the fundamental frequency band and a band corresponding to a frequency response characteristic of the sensor among the harmonic band.

5. The biological information detection device according to claim 2, wherein
   when a signal-to-noise ratio of the detection signal in an assumed heartbeat fundamental frequency band, which is a band in which the fundamental frequency is assumed to be present, is less than a second threshold, the estimation unit detects a peak of a frequency domain signal of the detection signal in an assumed heartbeat harmonic band, which is a band in which a frequency higher than the fundamental frequency is assumed to be present, and estimates the fundamental frequency based on a frequency of the peak detected in the assumed heartbeat harmonic band.

6. The biological information detection device according to claim 5, wherein
   the estimation unit detects a peak of a frequency domain signal of the detection signal in a band that is an integral multiple of the assumed heartbeat fundamental frequency band as the assumed heartbeat harmonic band, and estimates the fundamental frequency by performing a reverse calculation based on a difference in frequency of the peaks detected in the assumed heartbeat harmonic band.

7. The biological information detection device according to claim 5, wherein
the estimation unit detects, as respiration body motion information, a body motion due to respiration based on a frequency domain signal of the detection signal, and estimates a fundamental frequency of the body motion based on the detected respiration body motion information and the frequency of the peak detected in the assumed heartbeat fundamental frequency band or the assumed heartbeat harmonic band.

8. The biological information detection device according to claim 7, wherein

the estimation unit detects a fundamental frequency of respiration as the respiration body motion information, determines whether the detected fundamental frequency of the respiration and a respiration harmonic with respect to the fundamental frequency of the respiration overlap the frequency of the peak detected in the assumed heartbeat fundamental frequency band,
when the frequencies do not overlap, estimates the frequency of the peak detected in the assumed heartbeat fundamental frequency band as the fundamental frequency of the detection signal, and
when the frequencies overlap, detects a frequency of a peak that does not overlap the fundamental frequency of the respiration and the respiration harmonic from the assumed heartbeat fundamental frequency band and estimates the frequency of the peak as the fundamental frequency of the body motion.

9. The biological information detection device according to claim 2, wherein
with respect to each of first peaks that are peaks in the detection signal after the application of the filter, the detection unit detects an array of peaks of the detection signal as the biological information according to positions of a second peak that is a peak immediately before a time of the first peak in the detection signal before the application of the filter and a third peak that is a peak immediately after the time of the first peak in the detection signal before the application of the filter.

10. The biological information detection device according to claim 9, wherein
in each of the first peaks, when a position of the first peak is separated from a central position of an interval between the second peak and the third peak by a predetermined distance or greater, the detection unit sets one of the second peak and the third peak that is closer to the first peak as a peak of the detection signal, and detects the array of the peaks of the detection signal as the biological information.

11. The biological information detection device according to claim 10, wherein
when the position of the first peak is within the predetermined distance from the central position of the interval between the second peak and the third peak, the detection unit further detects the second peak as the peak of the detection signal, and detects the array of the peaks of the detection signal as the biological information.

12. The biological information detection device according to claim 9, wherein
the detection unit ignores a peak deviated from the fundamental frequency among the peaks of the detection signal after the application of the filter.

13. The biological information detection device according to claim 1, further comprising:
a correction unit configured to correct a position of the peak of the detection signal when the peak of the detection signal is present in a predetermined outlier range.

14. The biological information detection device according to claim 1, wherein
the sensor detects information on a heartbeat of the person as the information on the body motion of the person.

15. The biological information detection device according to claim 14, wherein
the sensor detects the information on the heartbeat by a pneumatic change of an air bag configured to press against the person while the person is seated.

## FIG. 1

# FIG. 2

## FIG. 3

**BIOLOGICAL INFORMATION DETECTION DEVICE** (111)

- PNEUMATIC SENSOR (12) → DETECTION SIGNAL → PROCESSOR (121)
  - CPU
  - RAM
  - ROM
  - SSD
  - I/F

→ BIOLOGICAL INFORMATION →

**VEHICLE CONTROL SYSTEM** (112)

- ECU (131)
  - DRIVE MECHANISM (132)
  - BRAKE MECHANISM (133)
  - STEERING MECHANISM (134)
  - USER I/F (135)

100

EP 4 623 815 A1

## FIG. 4

111

BIOLOGICAL INFORMATION DETECTION DEVICE

201

ACQUISITION UNIT

202

FREQUENCY ANALYSIS UNIT

203

NOISE LEVEL ESTIMATION UNIT

204

FUNDAMENTAL FREQUENCY ESTIMATION UNIT

205

FILTER PROCESSING UNIT

206

PEAK DETECTION UNIT

207

PEAK CORRECTION UNIT

208

OUTPUT UNIT

# FIG. 5

```
                    START

                      │  ⌐S101
                      ▼
        ┌─────────────────────────────┐
        │   DETECTION SIGNAL INPUT     │
        └─────────────────────────────┘
                      │  ⌐S102
                      ▼
        ┌─────────────────────────────┐
        │      FREQUENCY ANALYSIS      │
        └─────────────────────────────┘
                      │  ⌐S103
                      ▼
        ┌─────────────────────────────┐
        │    NOISE LEVEL ESTIMATION    │
        └─────────────────────────────┘
                      │  ⌐S104
                      ▼
        ┌─────────────────────────────┐
        │ FUNDAMENTAL FREQUENCY ESTIMATION │
        └─────────────────────────────┘
                      │  ⌐S105
                      ▼
        ┌─────────────────────────────┐
        │      FILTER PROCESSING       │
        └─────────────────────────────┘
                      │  ⌐S106
                      ▼
        ┌─────────────────────────────┐
        │       PEAK DETECTION         │
        └─────────────────────────────┘
                      │  ⌐S107
                      ▼
        ┌─────────────────────────────┐
        │       PEAK CORRECTION        │
        └─────────────────────────────┘
                      │  ⌐S108
                      ▼
        ┌─────────────────────────────┐
        │ BIOLOGICAL INFORMATION OUTPUT │
        └─────────────────────────────┘
                      │
                      ▼
                     END
```

## FIG. 6

EP 4 623 815 A1

## FIG. 7

START

S201
SN RATIO OF ASSUMED HEARTBEAT FUNDAMENTAL FREQUENCY BAND ≥ SECOND THRESHOLD?

No

Yes

S202
PEAK DETECTION WITHIN ASSUMED HEARTBEAT FUNDAMENTAL FREQUENCY BAND

S203
SET MAXIMUM PEAK FREQUENCY TO FUNDAMENTAL FREQUENCY

S204
CALCULATE AUTOCORRELATION COEFFICIENT

S205
PEAK DETECTION BASED ON AUTOCORRELATION COEFFICIENT OUTSIDE ASSUMED HEARTBEAT FUNDAMENTAL FREQUENCY BAND

S206
SET MOVEMENT AMOUNT IN FREQUENCY AXIS DIRECTION BASED ON AUTOCORRELATION COEFFICIENT TO FUNDAMENTAL FREQUENCY

RETURN

# FIG. 8

START

S301

EXTRACT, BASED ON FUNDAMENTAL FREQUENCY, HARMONIC BAND AT FREQUENCY f [Hz] OR LESS (SET ACCORDING TO SENSOR FREQUENCY CHARACTERISTIC)

S302

CALCULATE SN RATIO FOR EACH FUNDAMENTAL FREQUENCY BAND AND HARMONIC BAND

S303

GENERATE BANDPASS FILTER HAVING ONLY BAND WHERE SN RATIO ≥ FIRST THRESHOLD AS PASSBAND

S304

APPLY BANDPASS FILTER TO DETECTION SIGNAL

RETURN

# FIG. 9

START

S401
DETECT PEAK TIME OF SIGNAL
AFTER FILTER APPLICATION (ARRAY A)

S402
DETECT PEAK TIME OF SIGNAL
BEFORE FILTER APPLICATION (ARRAY B)

S403
EXTRACT ELEMENT OF ARRAY B
IMMEDIATELY BEFORE EACH ELEMENT
OF ARRAY A (ARRAY C)

S404
EXTRACT ELEMENT OF ARRAY B
IMMEDIATELY AFTER EACH ELEMENT
OF ARRAY A (ARRAY D)

S405
OBTAIN abs((A − C)/(D − C) − 0.5) FOR EACH
ELEMENT OF ARRAYS A, C, AND D, AND
CALCULATE AVERAGE VALUE d THEREOF

S406
d > THRESHOLD?

No

S408
SET ARRAY C AS PEAK ARRAY

Yes
S407
EXTRACT ELEMENT OF ARRAY B
CLOSEST TO EACH ELEMENT OF
ARRAY A AS PEAK ARRAY

RETURN

# FIG. 10

EP 4 623 815 A1

## FIG. 11

```
              ┌──────────┐
              │  START   │
              └────┬─────┘
                   │           S501
  ┌────────────────┴────────────────────┐
  │ CALCULATE DIFFERENCE BETWEEN EACH     │
  │ ELEMENT OF PEAK ARRAY AND IMMEDIATELY │
  │ PRECEDING ELEMENT (HEARTBEAT INTERVAL │
  │ ARRAY)                                │
  └────────────────┬────────────────────┘
                   │           S502
  ┌────────────────┴────────────────────┐
  │ DETECT OUTLIER FOR HEARTBEAT INTERVAL │
  │ ARRAY, AND STORE INDEX OF ELEMENT     │
  │ DETERMINED TO BE OUTLIER AND WHETHER   │
  │ IT IS ON UPPER SIDE OR LOWER SIDE IN   │
  │ ARRAY                                 │
  └────────────────┬────────────────────┘
                   │           S503
  ┌────────────────┴────────────────────┐
  │              1 → i                    │
  └────────────────┬────────────────────┘
```

- S504: i < NUMBER OF OUTLIERS? — No / Yes
- S505: DIFFERENCE BETWEEN i-TH AND (i + 1)-TH OUTLIER INDEXES < THRESHOLD? — No / Yes
- S506: i-TH OUTLIER ON UPPER SIDE AND (i + 1)-TH OUTLIER ON LOWER SIDE? — No / Yes
- S507: CHANGE ELEMENT OF PEAK ARRAY FROM i-TH INDEX TO (i + 1)-TH INDEX − 1 TO PEAK AT TIME IMMEDIATELY BEFORE ARRAY B
- S509: i-TH OUTLIER ON LOWER SIDE AND (i + 1)-TH OUTLIER ON UPPER SIDE? — No / Yes
- S510: CHANGE ELEMENT OF PEAK ARRAY FROM i-TH INDEX TO (i + 1)-TH INDEX − 1 TO PEAK AT TIME IMMEDIATELY AFTER ARRAY B
- S508: i+1 → i
- S511: i+1 → i
- RETURN

# FIG. 12

```
                    ┌─────────────┐
                    │    START    │
                    └──────┬──────┘
                           │
          ┌────────────────┴────────────────────────┐
          ▼ ⌐S101                                    ▼ ⌐S601
┌─────────────────────────┐          ┌─────────────────────────────┐
│  DETECTION SIGNAL INPUT │          │    DETECTION SIGNAL INPUT    │
└────────────┬────────────┘          │     DURING RESTING STATE     │
             │ ⌐S102                  └──────────────┬──────────────┘
             ▼                                       │ ⌐S602
┌─────────────────────────┐          ┌─────────────────────────────┐
│    FREQUENCY ANALYSIS   │          │  ACQUIRE, IN FREQUENCY DOMAIN, │
└────────────┬────────────┘          │   FUNDAMENTAL FREQUENCY OF   │
             │ ⌐S103                  │  RESPIRATION BASED ON HF BAND │
             ▼                        │     (0.04 Hz TO 0.15 Hz)     │
┌─────────────────────────┐          └──────────────┬──────────────┘
│   NOISE LEVEL ESTIMATION│                          │
└────────────┬────────────┘◄────────────────────────┘
             │ ⌐S604
┌─────────────────────────┐
│  FUNDAMENTAL FREQUENCY  │
│       ESTIMATION        │
└────────────┬────────────┘
             │ ⌐S105
┌─────────────────────────┐
│     FILTER PROCESSING   │
└────────────┬────────────┘
             │ ⌐S106
┌─────────────────────────┐
│      PEAK DETECTION     │
└────────────┬────────────┘
             │ ⌐S107
┌─────────────────────────┐
│      PEAK CORRECTION    │
└────────────┬────────────┘
             │ ⌐S108
┌─────────────────────────┐
│ BIOLOGICAL INFORMATION OUTPUT │
└────────────┬────────────┘
             │
       ┌─────▼──────┐
       │    END     │
       └────────────┘
```

## FIG. 13

```
                          START

                           │
                           ▼
                    ╱────────────╲              S201
                  ╱   SN RATIO OF   ╲
                ╱   ASSUMED HEARTBEAT  ╲    No
              ╱  FUNDAMENTAL FREQUENCY  ╲────────────────┐
                ╲    BAND ≥ SECOND      ╱                 │
                  ╲    THRESHOLD?      ╱                  │
                    ╲────────────╱                       ▼
                           │                                        S204
                      Yes  │  S202           ┌────────────────────────────────────┐
                           ▼                 │ CALCULATE AUTOCORRELATION COEFFICIENT│
   ┌──────────────────────────────┐         └────────────────────────────────────┘
   │  PEAK DETECTION WITHIN ASSUMED │                       │
   │HEARTBEAT FUNDAMENTAL FREQUENCY │                       ▼          S205
   │             BAND               │         ┌────────────────────────────────────┐
   └──────────────────────────────┘         │ PEAK DETECTION BASED ON AUTOCORRELATION│
                           │  S701           │ COEFFICIENT OUTSIDE ASSUMED HEARTBEAT  │
                           ▼                 │   FUNDAMENTAL FREQUENCY BAND           │
   ┌──────────────────────────────┐         └────────────────────────────────────┘
   │  EXTRACT CANDIDATE OF FUNDAMENTAL│                     │  S706
   │    FREQUENCY OF HEARTBEAT      │         ┌────────────────────────────────────┐
   └──────────────────────────────┘         │ SET MOVEMENT AMOUNT IN FREQUENCY AXIS  │
                           │                 │  DIRECTION BASED ON AUTOCORRELATION    │
                           │                 │ COEFFICIENT TO CANDIDATE OF FUNDAMENTAL│
                           │                 │             FREQUENCY                  │
                           │                 └────────────────────────────────────┘
                           │◄──────────────────────────────┘
                           │  S702
                           ▼
   ┌──────────────────────────────┐
   │  COMPARE CANDIDATE OF FUNDAMENTAL│
   │FREQUENCY OF HEARTBEAT (FREQUENCY AT│
   │ MAXIMUM PEAK) WITH FUNDAMENTAL │
   │  FREQUENCY OF RESPIRATION AND  │
   │     RESPIRATION HARMONIC       │
   └──────────────────────────────┘
                           │◄────────────────────────────────────────────────────┐
                           ▼                                                       │
                    ╱────────────╲   S703                                          │
                  ╱  RESPIRATION   ╲  n: 1, 2, 3, 4, 5 IS COMPARED IN ORDER         │
                ╱  (FUNDAMENTAL     ╲                                               │
               ╱    FREQUENCY        ╲                                             │
              ╱ AND RESPIRATION HARMONIC) Xn ╲  Yes                                │
              ╲  OVERLAP CANDIDATE OF ╱────────────────┐                           │
               ╲   FUNDAMENTAL      ╱                  ▼              S705          │
                ╲ FREQUENCY OF     ╱      ┌──────────────────────────────┐         │
                  ╲ HEARTBEAT?    ╱       │   SET PEAK FREQUENCY HAVING     │       │
                    ╲────────────╱        │ SECOND MAXIMUM VALUE AS CANDIDATE│      │
                           │              │    OF FUNDAMENTAL FREQUENCY     │       │
                      No   │  S704        └──────────────────────────────┘         │
                           ▼                                 └─────────────────────┘
   ┌──────────────────────────────┐
   │  SET CANDIDATE OF FUNDAMENTAL  │
   │FREQUENCY TO FUNDAMENTAL FREQUENCY│
   └──────────────────────────────┘
                           │
                           ▼
                       RETURN
```

**EUROPEAN SEARCH REPORT**

Application Number

EP 25 16 4351

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/102184 A1 (KONINKL PHILIPS NV [NL]) 30 June 2016 (2016-06-30) <br> * abstract * <br> * page 2, line 11 - line 16 * <br> * page 2, line 32 - page 3, line 4 * <br> * page 5, line 15 - line 31 * <br> * page 7, line 1 - line 18 * <br> * claim 1 * <br> ----- | 1-15 | INV. <br> A61B5/0205 <br> A61B5/00 <br> A61B5/024 <br> A61B5/08 <br> A61B5/113 <br> B60N2/00 |
| X | US 2020/317207 A1 (SLOUSHCH ILYA [IL] ET AL) 8 October 2020 (2020-10-08) <br> * abstract * <br> * paragraphs [0012], [0097] - [0103]; figures 5A-B * <br> ----- | 1-15 | |
| X | AMY DIANE DROITCOUR: "NON-CONTACT MEASUREMENT OF HEART AND RESPIRATION RATES WITH A SINGLE-CHIP MICROWAVE DOPPLER RADAR", <br> A DISSERTATION SUBMITTED TO THE DEPARTMENT OF ELECTRICAL ENGINEERING AND THE COMMITTEE ON GRADUATE STUDIES OF STANFORD UNIVERSITY IN PARTIAL FULFILLMENT OF THE REQUIREMENTS FOR THE DEGREE OF DOCTOR OF , <br> 1 June 2006 (2006-06-01), pages 1-470, XP002688022, <br> Retrieved from the Internet: <br> URL:http://72.52.208.92/~gbpprorg/mil/inter/Droitcour_Thesis.pdf <br> [retrieved on 2012-11-26] | 1,14 | |
| A | * abstract * <br> * sect.7.4.3; 7.5.1 * <br> ----- | 2-13,15 | |
| A | US 2006/283652 A1 (YANAI KENICHI [JP] ET AL) 21 December 2006 (2006-12-21) <br> * paragraph [0102]; claims 1-14; figures 1-12 * <br> ----- | 1-15 | |

TECHNICAL FIELDS
SEARCHED      (IPC)

A61B
B60N

-/--

The present search report has been drawn up for all claims

| Place of search | Date of completion of the search | Examiner |
|---|---|---|
| The Hague | 19 August 2025 | Delval, Christophe |

page 1 of 3

Europäisches
Patentamt

European
Patent Office

Office européen
des brevets

## EUROPEAN SEARCH REPORT

**Application Number**

EP 25 16 4351

### DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | MIZUNO NAOKI ET AL: "An Adaptive Filtering Technique for Driver's Heart Rate Monitoring through Vibration Signal by Seat-Embedded Piezoelectric Sensors", 5TH IFAC SYMPOSIUM ON MODELLING AND CONTROL IN BIOMEDICAL SYSTEMS 2003, MELBOURNE, AUSTRALIA, 21-23 AUGUST 2003, [Online] vol. 46, no. 11, 1 January 2013 (2013-01-01), pages 647-652, XP093305676, ISSN: 1474-6670, DOI: 10.3182/20130703-3-FR-4038.00136 Retrieved from the Internet: URL:https://www.sciencedirect.com/science/article/pii/S1474667016330178/pdf?md5=90cab19ec0b99c064c8cad7911c7d8e4&pid=1-s2.0-S1474667016330178-main.pdf> [retrieved on 2025-08-19] * abstract * * the whole document * | 1-15 | |
| A | US 2017/347961 A1 (PERRAUT JOHN M [US] ET AL) 7 December 2017 (2017-12-07) * abstract * * claims 1-9; figures 1-4 * * the whole document * | 1-15 | **TECHNICAL FIELDS SEARCHED (IPC)** |
| A | KR 2011 0119477 A (SAMSUNG ELECTRONICS CO LTD [KR]) 2 November 2011 (2011-11-02) * abstract * * the whole document * * claims 1-9; figures 1-3 * | 1-15 | |

-/--

The present search report has been drawn up for all claims

| Place of search | Date of completion of the search | Examiner |
|---|---|---|
| The Hague | 19 August 2025 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

                                                                    
& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 25 16 4351

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | DOS SANTOS LAURITA ET AL: "Application of an automatic adaptive filter for Heart Rate Variability analysis", MEDICAL ENGINEERING & PHYSICS, BUTTERWORTH-HEINEMANN, GB, [Online] vol. 35, no. 12, 1 December 2013 (2013-12-01), pages 1778-1785, XP028797576, ISSN: 1350-4533, DOI: 10.1016/J.MEDENGPHY.2013.07.009 Retrieved from the Internet: URL:https://www.sciencedirect.com/science/ article/pii/S1350453313001677/pdfft?md5=15 458738730bfcfc6c52f012440ecb43&pid=1-s2.0- S1350453313001677-main.pdf> [retrieved on 2013-08-18] * abstract * * the whole document * ----- | 1-15 | |
| | | | **TECHNICAL FIELDS SEARCHED (IPC)** |

The present search report has been drawn up for all claims

| Place of search | Date of completion of the search | Examiner |
|---|---|---|
| The Hague | 19 August 2025 | Delval, Christophe |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

 

& : member of the same patent family, corresponding document

EPO FORM 1503 03.82 (P04C01)

## ANNEX TO THE EUROPEAN SEARCH REPORT
## ON EUROPEAN PATENT APPLICATION NO.

EP 25 16 4351

This annex lists the patent family members relating to the patent documents cited in the above-mentioned European search report.
The members are as contained in the European Patent Office EDP file on
The European Patent Office is in no way liable for these particulars which are merely given for the purpose of information.

19-08-2025

| Patent document cited in search report | | Publication date | Patent family member(s) | | Publication date |
|---|---|---|---|---|---|
| WO 2016102184 | A1 | 30-06-2016 | NONE | | |
| US 2020317207 | A1 | 08-10-2020 | CA | 3071960 A1 | 07-02-2019 |
| | | | CN | 110944878 A | 31-03-2020 |
| | | | EP | 3661815 A1 | 10-06-2020 |
| | | | JP | 7093918 B2 | 01-07-2022 |
| | | | JP | 2020531063 A | 05-11-2020 |
| | | | KR | 20200033324 A | 27-03-2020 |
| | | | US | 2020317207 A1 | 08-10-2020 |
| | | | WO | 2019026076 A1 | 07-02-2019 |
| US 2006283652 | A1 | 21-12-2006 | JP | 2006346093 A | 28-12-2006 |
| | | | US | 2006283652 A1 | 21-12-2006 |
| US 2017347961 | A1 | 07-12-2017 | NONE | | |
| KR 20110119477 | A | 02-11-2011 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2009022638 A